(19) Europäisches Patentamt
European Patent Office
Office européen des brevets

(11) Veröffentlichungsnummer: **0 036 572**
**A1**

(12) **EUROPÄISCHE PATENTANMELDUNG**

(21) Anmeldenummer: **81101819.1**

(22) Anmeldetag: **12.03.81**

(51) Int. Cl.³: **C 07 D 207/16**, C 07 D 277/06, C 07 D 401/12, C 07 D 409/12, C 07 D 417/12, A 61 K 31/40, A 61 K 31/425, A 61 K 31/44

(30) Priorität: **22.03.80 DE 3011239**

(43) Veröffentlichungstag der Anmeldung: **30.09.81 Patentblatt 81/39**

(84) Benannte Vertragsstaaten: **AT BE CH DE FR IT LI LU NL SE**

(71) Anmelder: **C.H. BOEHRINGER SOHN, Postfach 200, D-6507 Ingelheim am Rhein (DE)**

(72) Erfinder: **Esser, Franz, Dr., Albrecht-Dürer-Strasse 24, D-6507 Ingelheim/Rhein (DE)**
Erfinder: **Roos, Otto, Dr., Elsheimer Strasse 36, D-6501 Schwabenheim (DE)**
Erfinder: **Lösel, Walter, Dr., Im Herzenacker 26, D-6535 Gau-Algesheim (DE)**
Erfinder: **Wiedemann, Ingrid, Dr., Ernst-Ludwig-Strasse 61, D-6535 Gau-Algesheim (DE)**
Erfinder: **Gaida, Wolfram, Dr., Paul-Clemen-Strasse 14, D-6507 Ingelheim/Rhein (DE)**
Erfinder: **Hoefke, Wolfgang, Dr., Rosselstrasse 21, D-6200 Wiesbaden (DE)**

(54) **Substituierte Alkylthioacylaminosäuren, Verfahren zu ihrer Herstellung und pharmazeutische Zusammensetzungen.**

(57) Alkylthioacylaminosäuren der allgemeinen Formel

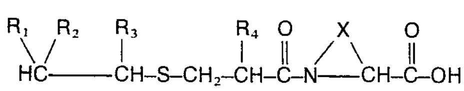 (I)

worin

$R_1$ die Nitro-, Cyano- oder Pyridylgruppe oder eine der Gruppen $-COR'$, $-COOR'$, $-PO(OR')_2$ oder

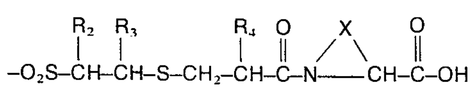 ,

$R_2$ Wasserstoff, eine niedere Alkylgruppe mit 1 oder 2 Kohlenstoffatomen, eine der Gruppen $-COOR'$, $COR'$ oder den Rest $-SO_2CH_3$,

$R_3$ Wasserstoff, eine Alkoxygruppe mit 1–3 Kohlenstoffatomen, eine Phenylgruppe oder einen Thienylrest,

$R_4$ Wasserstoff oder die Methylgruppe,

X eine der Gruppen $-CH_2-CH_2-CH_2-$ oder $-CH_2-S-CH_2-$

und

R' eine Alkylgruppe mit 1 oder 2 Kohlenstoffatomen bedeuten sowie ihre basischen Salze, besitzen blutdrucksenkende Wirkung, die auf einer Hemmung des Angiotensin-I-Converting-Enzyms beruht.

**EP 0 036 572 A1**

ACTORUM AG

COMPLETE DOCUMENT

Die Erfindung betrifft neue substituierte Alkylthioacylaminosäuren der allgemeinen Formel

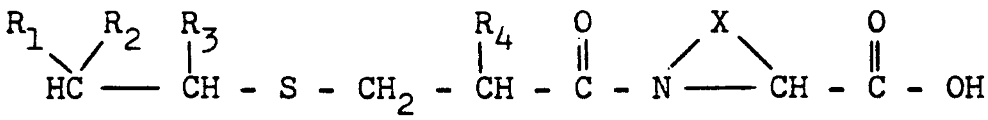 (I)

sowie ihre basischen Salze.

In dieser Formel bedeuten:

$R_1$ die Nitro-, Cyano- oder Pyridylgruppe oder eine der Gruppen - COR', - COOR', - $PO(OR')_2$ oder

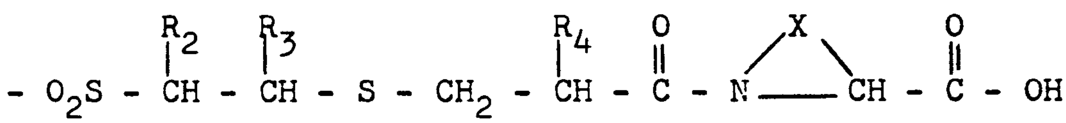 ,

$R_2$ Wasserstoff, eine niedere Alkylgruppe mit 1 oder 2 Kohlenstoffatomen, eine der Gruppen - COOR', COR' oder den Rest - $SO_2CH_3$,

$R_3$ Wasserstoff, eine Alkoxygruppe mit 1 - 3 Kohlenstoffatomen, eine Phenylgruppe oder einen Thienylrest,

$R_4$ Wasserstoff oder die Methylgruppe,

X eine der Gruppen - $CH_2$ - $CH_2$ - $CH_2$ - oder - $CH_2$ - S - $CH_2$ - und

R' eine Alkylgruppe mit 1 oder 2 Kohlenstoffatomen.

Die als Bedeutung für den Rest $R_1$ genannte Pyridylgruppe kann in 2-, 3- oder 4-Stellung mit dem übrigen Molekül verknüpft sein; bevorzugt ist die 4-Stellung.

Die neuen Verbindungen weisen im allgemeinen mehrere Asymmetriezentren auf und liegen daher als Diastereoisomere oder in Form ihrer Racemate beziehungsweise ihrer racemischen Gemische vor. Die Erfindung umfaßt sowohl die racemischen Gemische als auch die einzelnen Diastereomerenpaare. Bevorzugt sind diejenigen Enantiomeren, bei denen das asymmetrische C-Atom der Aminosäure in L-Konfiguration vorliegt.

Die erfindungsgemäßen Verbindungen bilden Salze mit anorganischen und organischen Basen. Beispiele für anorganische Basen sind Ammoniak, Alkalimetallhydroxyde wie Natrium- oder Kaliumhydroxyd oder Erdalkalimetallhydroxyde wie Calcium- oder Magnesiumhydroxyd; an organischen Basen seien genannt Dicyclohexylamin, N,N'-Dibenzyläthylendiamin, N,N'-Bis-(dehydroabietyl)-äthylendiamin, N-Methyl-D-glycamin, Arginin oder Lysin.

Die neuen Stoffe können nach verschiedenen Verfahren erhalten werden:

a) Durch Addition der freien Mercaptogruppe einer Verbindung der allgemeinen Formel

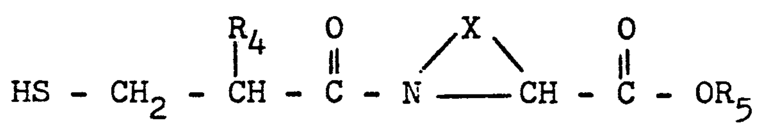

(II)

worin die Reste $R_4$ und X die oben angegebene Bedeutung haben und $R_5$ Wasserstoff oder eine niedere Alkylgruppe mit 1 - 3 Kohlenstoffatomen bedeutet,
an die Doppelbindung eines durch mindestens eine elektronenanziehende Gruppe aktivierten Olifins der allgemeinen Formel

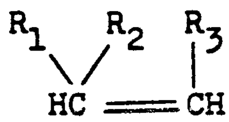

(III)

worin die Reste $R_1$, $R_2$ und $R_3$ die oben angegebene Bedeutung haben,
und, falls $R_5$ von Wasserstoff verschieden ist, anschließende saure oder alkalische Abspaltung der Estergruppe.

Die Additionsreaktion kann in Gegenwart oder Abwesenheit von Lösungsmitteln durchgeführt werden. Geeignete Lösungsmittel sind Wasser, Alkohol, Äther, Tetrahydrofuran, Dioxan, halogenierte Kohlenwasserstoffe wie Dichlormethan, Benzol, Toluol, Essigester, Aceton, Dimethylsulfoxyd, Dimethylformamid und Pyridin. Auch das eingesetzte aktivierte Olefin kann, falls es flüssig ist, als Lösungsmittel verwendet werden.
Bevorzugte Lösungsmittel sind Wasser und Äthanol.

Die Addition kann radikalisch oder ionisch geführt werden. Die bevorzugte ionisch geführte Addition wird in einem pH-Bereich von 6 bis 14, vorzugsweise zwischen pH 7 und 10, durchgeführt. Der Zusatz eines oder mehrerer basischer Stoffe wie Ammoniak,Triäthylamin,Trimethylamin,Dicyclohexylamin, N-Methylmorpholin, Piperidin, Pyridin, Trimethylbenzylammoniumhydroxyd, Alkalihydroxyd, Alkalicarbonat oder Alkalialkoholat hat sich dabei als günstig erwiesen. Bevorzugte Basen sind Ammoniak, Triäthylamin, Dicyclohexylamin und N-Methylmorpholin.

Die Reaktionstemperatur bei der ionisch geführten Addition kann, je nach den eingesetzten Ausgangsstoffen, in weiten Grenzen schwanken und zwischen -80°C und +150°C liegen; bevorzugt wird ein Temperaturbereich zwischen 0 und 50°C.
Die Reaktion wird im allgemeinen unter Normaldruck und Stickstoffatmosphäre durchgeführt; die Anwendung von erhöhtem Druck kann jedoch in manchen Fällen von Vorteil sein.

Bei Durchführung der radikalisch geführten Addition ist ein Radikalbildner einzusetzen, beispielsweise Benzoylperoxyd, Di-tert.-butylperoxyd, Ascaridol, Azobis-(isobutyronitril) oder Kaliumpersulfat.
Die Reaktion erfolgt unter den bei der ionischen Addition bereits erwähnten Bedingungen.

b) Ein weiteres Verfahren zur Herstellung der neuen Verbindungen besteht in der Umsetzung einer substituierten Alkylthiocarbonsäure der allgemeinen Formel

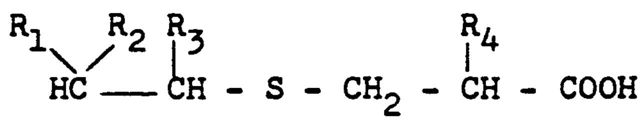

(IV)

worin die Reste $R_1$, $R_2$, $R_3$ und $R_4$ die oben erwähnte Bedeutung haben,
mit einer Aminosäure der allgemeinen Formel

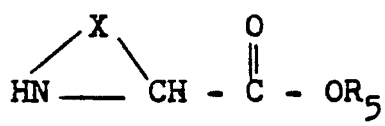

(V)

worin $R_5$ die oben erwähnte Bedeutung hat.

Die Verknüpfung der Verbindungen der allgemeinen Formeln IV und V erfolgt mittels in der Peptidchemie üblichen Kupplungsreaktionen unter Verwendung einer aktivierten Alkylthiocarbonsäure der Formel IV; als carboxylaktivierende Gruppen kommen in Frage Säurechloride, Azide, Anhydride, p-Nitrophenylester, Trichlorphenylester, Thiophenylester, o-Cyanomethylester usw.. Als Kupplungsmittel werden bevorzugt Carbonyldiimidazol, Dicyclohexylcarbodiimid oder Chlorameisensäureester verwendet (vgl. Houben-Weyl, Methoden der organischen Chemie, Band XV, Teil II, 1974).
Eine eventuell vorhandene Estergruppe ($R_5$ = O-Alkyl) wird im Anschluß an die Kupplungsreaktion durch hydrolytische oder acidolytische Spaltung entfernt.

c) Dimere Verbindungen der allgemeinen Formel I, worin $R_1$ die Gruppe

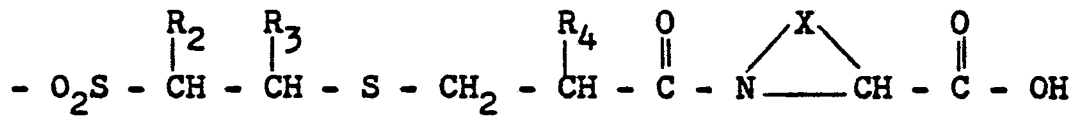

bedeutet, erhält man durch Umsetzung einer Verbindung der allgemeinen Formel II mit Divinylsulfon im Molverhältnis 2:1 unter den bei a) und b) angegebenen Reaktionsbedingungen.

Bei den vorstehend beschriebenen Verfahren können die Ausgangsverbindungen in Form ihrer racemischen Gemische oder ihrer Diastereoisomeren vorliegen. Liegen die racemischen Gemische zugrunde, können die erhaltenen Reaktionsprodukte durch die üblichen Verfahren, beispielsweise durch fraktionierte Kristallisation oder nach dem üblichen chromatographischen Verfahren, gespalten werden.

Bevorzugt sind solche Verbindungen der allgemeinen Formel I, in denen $R_1$ Wasserstoff, Acetyl oder Methoxycarbonyl, $R_2$ Nitro, Acetyl oder Methoxycarbonyl, $R_3$ Phenyl, Thienyl oder Methoxy und $R_4$ Methyl bedeutet und X die oben genannte Bedeutung hat.
Als besonders bevorzugt seien das 1-[3-(1-Phenyl-2-nitroäthylthio)-2-D-methylpropanoyl]-L-prolin, das 1-[3-(1-Thienyl(2)-2-nitroäthylthio)-2-D-methylpropanoyl]-L-prolin, das 1-[3-(1-Methoxy-2,2-bis- methoxycarbonyl -äthylthio)-2-D-methylpropanoyl]-L-prolin, das 1-[3-(1-Phenyl-2-nitroäthylthio)-2-D,L-methylpropanoyl]-L-prolin und das 1-[3-(1-Phenyl-2,2-diacetyl-äthylthio)-2-D-methylpropanoyl]-L-prolin beziehungsweise deren Salze genannt.
Bevorzugt sind ferner Verbindungen, in denen die 3-Mercapto-2-methylpropionsäure in der D-Form und die der Formel I zugrundeliegende Aminosäure in der L-Form vorliegt.

Nach den vorstehend beschriebenen Verfahren können beispielsweise die folgenden Endprodukte erhalten werden:

1-[3-(1-Phenyl-2-nitroäthylthio)-2-D-methylpropanoyl]-L-prolin,

1-[3-(1-Thienyl(2)-2-nitroäthylthio)-2-D-methylpropanoyl]-L-prolin,

1-[3-(1-Phenyl-2-nitroäthylthio)-2-D,L-methylpropanoyl]-L-thiazolidin-4-carbonsäure,

1-[3-(1-Thienyl(3)-2-nitroäthylthio)-propanoyl]-L-prolin,

1-[3-(1-Phenyl-2,2-diacetyl-äthylthio)-2-D-methylpropanoyl]-L-prolin,

1-[3-(2-Methyl-2-nitrilo-äthylthio)-2-D-methylpropanoyl]-L-prolin,

1-[3-(2-Pyridyl(4)-äthylthio)-2-D-methylpropanoyl]-L-prolin,

1-[3-(1-Phenyl-2,2-bis-{äthoxycarbonyl}-äthylthio)-propanoyl]-L-prolin,

1-[3-(3-Oxo-n-butylthio)-2-D-methylpropanoyl]-L-prolin,

1-[3-(1-p-Cyanophenyl-2-nitro-äthylthio)-2-D-methylpropanoyl]-L-prolin,

1-[3-(1-p-Tolyl-2-nitro-äthylthio)-2-D-methylpropanoyl]-L-prolin,

1-[3-(1-Thienyl(3)-2-nitro-äthylthio)-propanoyl]-L-prolin,

1-[3-(1-Phenyl-2,2-diacetyl-äthylthio)-2-D-methylpropanoyl]-L-prolin,

1-[3-(1-Phenyl-2,2-bis-{äthoxycarbonyl}-äthylthio)-2-D-methylpropanoyl]-L-prolin,

1-[3-(1-Methoxy-2,2-bis-{methoxycarbonyl}-äthylthio)-2-D-methylpropanoyl]-L-prolin,

1-[3-(2-{O,O-Diäthylphosphonyl}-äthylthio)-2-D-methylpropanoyl]-L-prolin,

1-[3-(1-Phenyl-2-methylsulfonyl-äthylthio)-2-D-methylpropanoyl]-L-prolin,

1-[3-(1-Methoxy-2,2-bis-{methoxycarbonyl}-äthylthio)-2-D-methylpropanoyl]-L-prolin,

1-[3-(2-Äthoxycarbonyl-äthylthio)-2-D-methylpropanoyl]-L-prolin,

Sulfonyl-bis-[(äthylthio-2-D-methylpropanoyl)-L-prolin] der Formel

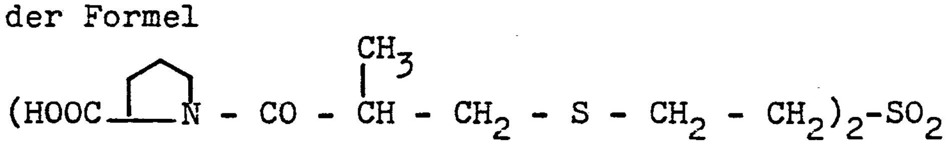 ,

Sulfonyl-bis-[(äthylthio-propanoyl)-L-prolin],

1-[3-(1-Phenyl-2-methylsulfonyl-äthylthio)-2-D-methylpropanoyl]-L-prolin.

Die Ausgangsverbindungen der allgemeinen Formeln II, III und V sind literaturbekannt. Verbindungen der allgemeinen Formel IV sind neu; sie können erhalten werden durch Addition der freien SH-Gruppe einer Mercaptoalkylcarbonsäure an die Doppelbindung eines durch eine oder mehrere elektronegative Gruppen substituierten Olefins.

Die substituierten Alkylthioacylaminosäuren der allgemeinen Formel I besitzen eine starke, lang anhaltende blutdrucksenkende Wirkung. Diese beruht auf einer Hemmung des Angiotensin I Converting Enzyms und damit einer Blockierung der Bildung des Vasokonstriktors Angiotensin II aus Angiotensin I. Darüber hinaus wirken die neuen Verbindungen hemmend auf das für den Bradykininabbau verantwortliche Enzym Kininase II, das als identisch mit dem oben genannten Converting Enzym gilt. Da Bradykinin eine gefäßerweiternde Wirkung besitzt, wird der blutdrucksenkende Effekt durch diese zusätzliche Wirkung verstärkt. Die durch Bradykinin erzeugte Blutdrucksenkung an normalen Ratten wird durch die neuen Verbindungen verstärkt. Ausdruck dieser Wirkung könnte auch die an unvorbehandelten genetischen Hochdruckratten beobachtete blutdrucksenkende Wirkung sein.

So erzeugt das Endprodukt nach Beispiel 1 an der genetischen Hochdruckratte eine Blutdrucksenkung von 19 % in einer Dosierung von 30 mg/kg p.o..
Die bei Ratten durch i.v.-Injektion von 3,0 $\mu$ g/kg Bradykinin verursachte Blutdrucksenkung wird durch eine Gabe von 1,38 $\mu$Mol/kg i.v. um 142 % verstärkt.

Die nach Beispiel 1 beziehungsweise 3 erhaltenen Endprodukte zeigen am Angiotensin-I-Converting-Enzym in vitro Hemmaktivitäten ($IC_{50}$) von 1 · $10^{-8}$ beziehungsweise 1,8 · $10^{-9}$ M/l. Als $IC_{50}$ wird hierbei diejenige Hemmstoffkonzentration bezeichnet, bei der das Angiotensin-I-Converting-Enzym zu 50 % gehemmt wird (s. H. S. Cheung, D. W. Cushman, Biochem. Biophys. Acta 293, 451 (1973)).

Den erfindungsgemäßen Verbindungen fehlt die SH-Gruppe, die bei Stoffen ähnlicher Struktur, beispielsweise bei Captopril, für einen großen Teil der Nebenwirkungen verantwortlich gemacht wird. Es kann daher erwartet werden, daß sich die neuen Verbindungen als besser verträglich erweisen.

Für die Anwendung in der Therapie werden die neuen Verbindungen mit üblichen pharmazeutischen Füll- oder Trägerstoffen, Streck-, Spreng-, Binde-, Gleit-, Dickungs- oder Verdünnungsmitteln gemischt. Als pharmazeutische Zubereitungsformen kömmen zum Beispiel Tabletten, Kapseln, Zäpfchen, Lösungen, Säfte, Emulsionen oder dispersible Pulver in Frage, wobei gewünschtenfalls weitere bekannte Wirkstoffe, z.B. Saluretica, Diuretica und/oder Antihypertonica zugefügt werden können.

Entsprechende Tabletten können beispielsweise durch Mischen des oder der Wirkstoffe mit bekannten Hilfsstoffen, beispielsweise inerten Verdünnungsmitteln, wie Calciumcarbonat, Calciumphosphat oder Milchzucker, Sprengmitteln, wie Maisstärke oder Alginsäure, Bindemitteln, wie Stärke oder Gelatine, Schmiermitteln, wie Magnesiumstearat oder Talk, und/oder Mitteln zur Erzielung des Depoteffektes, wie Carboxypolymethylen, Carboxymethylcellulose, Celluloseacetatphthalat, oder Polyvinylacetat erhalten werden. Die Tabletten können auch aus mehreren Schichten bestehen.

Entsprechend können Dragees durch Überziehen von analog den Tabletten hergestellten Kernen mit üblicherweise in Drageeüberzügen verwendeten Mitteln, beispielsweise Kollidon oder Schellack, Gummi arabicum, Talk, Titandioxid oder Zucker, hergestellt werden. Zur Erzielung eines Depoteffektes oder zur Vermeidung von Inkompatibilitäten kann der Kern auch aus mehreren Schichten bestehen. Desgleichen kann auch die Drageehülle zur Erzielung eines Depoteffektes aus mehreren Schichten bestehen, wobei die oben bei den Tabletten erwähnten Hilfsstoffe verwendet werden können.

Säfte der erfindungsgemäßen Wirkstoffe beziehungsweise Wirkstoffkombinationen können zusätzlich noch ein Süßungsmittel, wie Saccharin, Cyclamat, Glycerin oder Zucker sowie ein geschmacksverbesserndes Mittel, z.B. Aromastoffe, wie Vanillin oder Orangenextrakt, enthalten. Sie können außerdem Suspendierhilfsstoffe oder Dickungsmittel, wie Natriumcarboxymethylcellulose, Netzmittel, beispielsweise Kondensationsprodukte von Fettalkoholen mit Äthylenoxid, oder Schutzstoffe, wie p-Hydroxybenzoate, enthalten.

Injektionslösungen werden in üblicher Weise, z.B. unter Zusatz von Konservierungsmitteln, wie p-Hydroxybenzoaten, oder Stabilisatoren, wie Alkalisalzen der Äthylendiamintetraessigsäure und unter Zugabe geeigneter Lösungsvermittler hergestellt und in Injektionsflaschen oder Ampullen abgefüllt.

Die einen oder mehrere Wirkstoffe beziehungsweise Wirkstoffkombinationen enthaltenden Kapseln können beispielsweise hergestellt werden, indem man die Wirkstoffe mit inerten Trägern, wie Milchzucker oder Sorbit, mischt und in Gelatinekapseln einkapselt.

Die tägliche Dosis für die Anwendung der erfindungsgemäßen Verbindungen der allgemeinen Formel I beziehungsweise ihre Salze soll 50 - 1000, vorzugsweise 100 - 600 mg betragen und kann in 1 - 4 Einzeldosen verabreicht werden.

Die folgenden Beispiele dienen zur näheren Erläuterung der Erfindung:

Beispiel 1

1-[3-(1-Phenyl-2-nitro-äthylthio)-2-D-methylpropanoyl]-L-prolin

2,7 g 1-(3-Mercapto-2-D-methylpropanoyl)-L-prolin-tert.-butylester und 1,5 g ω-Nitrostyrol werden in 20 ml wasserfreiem Äthanol gelöst und unter Stickstoff bei Raumtemperatur mit 1 ml N-Methylmorpholin versetzt. Nach ca. 2 Stunden Stehenlassen bei Raumtemperatur wird das Lösungsmittel unter vermindertem Druck abgezogen und der Rückstand in Essigester aufgenommen. Die Essigesterphase wird zweimal mit $KHSO_4$-Lösung und einmal mit NaCl-Lösung ausgeschüttelt, mit $MgSO_4$ getrocknet und im Vakuum eingeengt. Das erhaltene Öl wird bei Raumtemperatur über eine Stunde mit ca. 30 ml 1n HCl in Eisessig behandelt, das Lösungsmittel am Rotationsverdampfer abgezogen und der Rückstand mehrmals mit Äther versetzt und eingedampft.
Man erhält 2 g (= 56 % d.Th.) der Titelverbindung als farbloses Öl.

Analyse: $C_{17}H_{22}N_2O_5S$

| | C | H | N | S |
|---|---|---|---|---|
| Berechnet: | 55,72 % | 6,05 % | 7,64 % | 8,75 % |
| Gefunden: | 56,09 % | 6,31 % | 7,43 % | 8,09 % |

Beispiel 2

1-[3-(2-Äthoxycarbonyl-äthylthio)-2-D-methylpropanoyl]-L-prolin

8,5 g 1-(3-Mercapto-2-D-methylpropanoyl)-L-prolin-tert.-butylester werden in 30 g Acrylsäureäthylester gelöst, mit 2,6 g Piperidin und 10 Tropfen Triton B versetzt und 5 Stunden unter Rückfluß gekocht.

Nach dem Abdestillieren des überschüssigen Acrylsäureesters am Rotationsverdampfer wird der Rückstand durch Chromatographie auf einer Kieselgelsäule mit dem Elutionsmittel $CH_2Cl_2$/Methanol (19:1) gereinigt. Das so erhaltene Öl wird mit ca. 100 ml 1n HCl in Eisessig übergossen und 1 Stunde bei 20°C gerührt. Nach dem Einengen und Abdampfen des Rückstandes mit Äther erhält man 3,2 g der Titelverbindung als Öl, das in wenig Äther gelöst, mit der äquivalenten Menge Dicyclohexylamin versetzt und mehrere Tage im Kühlschrank belassen wird. Durch Absaugen der abgeschiedenen Kristalle, Waschen mit Äther und Trocknen im Exsikkator können 3,5 g (= 23 % d.Th.) des Dicyclohexylammoniumsalzes der Titelverbindung isoliert werden, das pastige Konsistenz besitzt.

Analyse: $C_{26}H_{46}N_2O_5S$

| | C | H | N | S |
|---|---|---|---|---|
| Berechnet: | 62,62 % | 9,30 % | 5,62 % | 6,43 % |
| Gefunden: | 61,89 % | 8,81 % | 5,39 % | 6,48 % |

Beispiel 3

1-[3-(1-Thienyl(2)-2-nitro-äthylthio)-2-D-methylpropanoyl]-L-prolin

0,35 g 1-(3-Mercapto-2-D-methylpropanoyl)-L-prolin und 0,25 g ω-Nitro-2-vinyl-thiophen werden in wasserfreiem Äthanol gelöst, unter Stickstoff mit 0,16 ml N-Methylmorpholin versetzt und die Mischung über Nacht bei Raumtemperatur gerührt. Nach Einengen und Ansäuern des Reaktionsgemisches mit 2 n HCl wird mit Essigester extrahiert, der Essigesterauszug mit $NaHCO_3$-Lösung ausgeschüttelt und diese Lösung nach Ansäuern mit 2 n HCl erneut mit Essigester extrahiert.

Die so erhaltene organische Phase wird über $MgSO_4$ getrocknet und eingeengt. Das zurückbleibende Öl wird mit der äquivalenten Menge Dicyclohexylamin versetzt und mit Petroläther verrieben. Auf diese Weise erhält man 0,3 g (= 34 % d.Th.) des Dicyclohexylammoniumsalzes des 1-[3-(1-Thienyl(2)-2-nitro-äthylthio)-2-D-methylpropanoyl]-L-prolin in kristalliner Form vom Fp.: 61°C.

Analyse: $C_{27}H_{43}N_3O_5S_2$

| | C | H | N | S |
|---|---|---|---|---|
| Berechnet: | 58,56 % | 7,83 % | 7,59 % | 11,58 % |
| Gefunden: | 58,56 % | 7,95 % | 7,33 % | 11,85 % |

## Beispiel 4

### 1-[3-(1-Thienyl(2)-2-nitro-äthylthio)-2-D-methylpropanoyl]-L-prolin

2 g ω-Nitro-2-vinyl-thiophen und 1,55 g 3-Mercapto-2-D-methylpropionsäure werden in wasserfreiem Äthanol gelöst, unter Stickstoff mit 1,3 ml N-Methylmorpholin versetzt und die Mischung über Nacht bei Raumtemperatur gerührt. Nach dem Einengen wird der Rückstand in verdünnter Salzsäure aufgenommen, die wässrige Phase mit Essigester extrahiert, der Essigesterauszug mit $NaHCO_3$-Lösung ausgeschüttelt und diese Lösung nach Ansäuern mit 2 n HCl erneut mit Essigester extrahiert. Nach dem Trocknen der Lösung über $MgSO_4$ und Einengen werden 2,5 g 3-(1-Thienyl(2)-2-nitro-äthylthio)-2-D-methylpropionsäure als Öl erhalten. Das Öl wird in wasserfreiem Methylenchlorid gelöst, mit 0,9 ml N-Methylmorpholin versetzt, unter Feuchtigkeitsausschluß auf -15°C gekühlt und mit 1,2 g Chlorameisensäureisobutylester versetzt.

Nach 8 Minuten wird eine Lösung von 1,4 g L-Prolin-tert.-butylester in wasserfreiem Methylenchlorid zugegeben. Man läßt die Mischung auf Raumtemperatur kommen und rührt über Nacht. Danach wird die Lösung dreimal mit $NaHCO_3$-Lösung, mit gesättigter $KHSO_4$-Lösung und einmal mit destilliertem Wasser ausgeschüttelt, getrocknet und eingeengt, wobei ein Öl zurückbleibt. Das Öl wird 1 Stunde lang mit 1 n HCl in Eisessig bei Raumtemperatur behandelt. Danach wird es eingeengt, der Rückstand mehrmals mit Äther übergossen und das Lösungsmittel abgedampft. Das zurückbleibende Öl wird mit der äquivalenten Menge Dicyclohexylamin versetzt und mit Äther verrieben, wobei 2,8 g (= 42 % Gesamtausbeute) 1-[3-(1-Thienyl(2)-2-nitro-äthylthio)-2-D-methylpropanoyl]-L-prolin-dicyclohexylammoniumsalz in Form weißer Kristalle vom Fp.: 61°C erhalten werden.

Beispiel 5

3-[3-(1-Phenyl-2-nitro-äthylthio)-2-D,L-methylpropanoyl]-L-thiazolidin-4-carbonsäure

0,85 g 3-(3-Mercapto-2-methylpropanoyl)-L-thiazolidin-4-carbonsäure werden mit 0,5 g ω-Nitrostyrol, wie in Beispiel 3 beschrieben, umgesetzt. Man erhält 300 mg des 3-[3-(1-Phenyl-2-nitro-äthylthio)-2-D,L-methylpropanoyl]-L-thiazolidin-4-carbonsäure-dicyclohexylammoniumsalzes vom Fp.: 190°C (Zers.).

Analyse: $C_{28}H_{43}N_3O_5S_2$

| | C | H | N |
|---|---|---|---|
| Berechnet: | 59,44 % | 7,66 % | 7,43 % |
| Gefunden: | 59,49 % | 7,73 % | 7,05 % |

## Beispiel 6

### 1-[3-(2-Cyano-propylthio)-2-D-methylpropanoyl]-L-prolin

0,35 g 1(3-Mercapto-2-D-methylpropanoyl)-L-prolin werden in Wasser gelöst, die Lösung mit verdünntem Ammoniak auf pH 8,1 eingestellt und unter Stickstoff mit 0,2 g Methacrylnitril versetzt. Das Reaktionsgemisch wird über Nacht gerührt, das Wasser unter vermindertem Druck abdestilliert, der ölige Rückstand mit einem Äquivalent Dicyclohexylamin versetzt und mit Äther verrieben. Man erhält 0,4 g (= 53 % d.Th.) weiße Kristalle des Dicyclohexylammoniumsalzes der Titelverbindung vom Fp.: 125°C.

Analyse: $C_{25}H_{43}N_3O_3S$

| | C | H | N | S |
|---|---|---|---|---|
| Berechnet: | 64,48 % | 9,31 % | 9,02 % | 6,89 % |
| Gefunden: | 64,10 % | 9,51 % | 8,79 % | 7,05 % |

## Beispiel 7

### 1-[3-(2-Pyridyl(4)-äthylthio)-2-D-methylpropanoyl]-L-prolin

0,35 g 1-(3-Mercapto-2-D-methylpropanoyl)-L-prolin werden in Wasser gelöst, mit Triäthylamin auf pH 7 gestellt und unter Stickstoff mit 0,2 g 4-Vinylpyridin versetzt. Man rührt über Nacht und extrahiert danach zur Abtrennung von überschüssigem Vinylpyridin dreimal mit Essigester. Die wässrige Phase wird eingeengt, der Rückstand mit einem Äquivalent Dicyclohexylamin versetzt und mit Petroläther (Kp. 40-60°C) verrieben. Man erhält 0,4 g (= 50 % d.Th.) 1-[3-(2-Pyridyl(4)-äthylthio)-2-D-methylpropanoyl]-L-prolin-dicyclohexylammoniumsalz vom Fp.: 121°C.

Analyse: $C_{28}H_{45}N_3O_3S$

| | C | H | N | S |
|---|---|---|---|---|
| Berechnet: | 66,76 % | 9,00 % | 8,34 % | 6,37 % |
| Gefunden: | 66,54 % | 8,61 % | 8,06 % | 6,74 % |

Beispiel 8

1-[3-(1-p-Cyanophenyl-2-nitroäthylthio)-2-D-methyl-propanoyl]-L-prolin

3 g 1-(3-Mercapto-2-D-methylpropanoyl)-L-prolin und 1,3 g p-Cyano-Ω-nitrostyrol werden in Äthanol gelöst und unter Stickstoff mit 0,75 g N-Methylmorpholin versetzt. Man rührt über Nacht bei Raumtemperatur, destilliert das Äthanol ab und nimmt den Rückstand in gesättigter $KHSO_4$-Lösung auf. Die so entstehende Emulsion wird dreimal mit Essigester extrahiert und die vereinigten Essigester-Phasen dreimal mit 10 %iger $KHCO_3$-Lösung ausgeschüttelt. Die vereinigten $KHCO_3$-Phasen werden abgekühlt, mit konzentrierter Salzsäure angesäuert und wiederum dreimal mit Essigester extrahiert. Die vereinigten Essigester-Phasen werden mit gesättigter Kochsalzlösung gewaschen, mit $MgSO_4$ getrocknet und eingeengt. Der Rückstand wird in Isopropanol aufgenommen und mit Äther versetzt. Die ausfallenden Kristalle werden in Methanol aufgenommen, gekocht und eingeengt. Durch Lösen in Essigester und Eintropfen in Äther/Petroläther(40 - 60°C, 1:1) wird die Titelverbindung in kristalliner Form erhalten.
Ausbeute: 2,1 g = 72 % d.Th.; Fp. 78 - 80°C.

| Analyse: | $C_{18}H_{21}N_3O_5S$ | | | |
|---|---|---|---|---|
| | C | H | N | S |
| Berechnet: | 55,23 % | 5,41 % | 10,73 % | 8,19 % |
| Gefunden: | 55,37 % | 5,52 % | 9,60 % | 8,78 % |

Analog Beispiel 6 wurden ferner die folgenden Endprodukte erhalten:

1-[3-(1-Thienyl(3)-2-nitro-äthylthio)-propanoyl]-L-prolin, Dicyclohexylammoniumsalz. (hygroskopisch)

Analyse: $C_{26}H_{41}N_3O_5S_2$

| | C | H | N | S |
|---|---|---|---|---|
| Berechnet: | 57,86 % | 7,66 % | 7,78 % | 11,88 % |
| Gefunden: | 58,68 % | 7,86 % | 7,01 % | 10,79 % |

1-[3-(1-Phenyl-2,2-diacetyl-äthylthio)-2-D-methylpropanoyl]-L-prolin, Dicyclohexylammoniumsalz.
Fp.: 144 - 145°C
Analyse: $C_{33}H_{50}N_2O_5S$

| | C | H | N | S |
|---|---|---|---|---|
| Berechnet: | 67,54 % | 8,59 % | 4,77 % | 5,46 % |
| Gefunden: | 66,84 % | 8,65 % | 4,99 % | 5,53 % |

1-[3-(1-Phenyl-2,2-bis-{äthoxycarbonyl}-äthylthio)-2-D-methylpropanoyl]-L-prolin, Dicyclohexylammoniumsalz.
Fp.: 76 °C
Analyse: $C_{35}H_{54}N_2O_7S$

| | C | H | N | S |
|---|---|---|---|---|
| Berechnet: | 64,99 % | 8,41 % | 4,33 % | 4,96 % |
| Gefunden: | 66,30 % | 8,95 % | 4,24 % | 4,20 % |

1-[3-(1-Methoxy-2,2-bis-{methoxycarbonyl}-äthylthio)-2-D-methylpropanoyl]-L-prolin, Dicyclohexylammoniumsalz.

Fp.: 103°C

Analyse: $C_{28}H_{48}N_2O_8S$

| | C | H | N | S |
|---|---|---|---|---|
| Berechnet: | 58,72 % | 8,45 % | 4,89 % | 5,59 % |
| Gefunden: | 62,02 % | 8,47 % | 5,15 % | 4,96 % |

1-[3-(2-{O,O-Diäthylphosphonyl}-äthylthio)-2-D-methylpropanoyl]-L-prolin, Dicyclohexylammoniumsalz.

Fp.: 117°C

Analyse: $C_{27}H_{51}N_2O_6PS$

| | C | H | N | S |
|---|---|---|---|---|
| Berechnet: | 57,63 % | 9,15 % | 4,98 % | 5,70 % |
| Gefunden: | 58,15 % | 9,46 % | 5,25 % | 5,10 % |

Sulfonyl-bis-[(äthylthio-2-D-methylpropanoyl)-L-prolin], Bis-dicyclohexylammoniumsalz.

Fp.: 127°C

Analyse: $C_{46}H_{82}N_4O_8S_3$

| | C | H | N | S |
|---|---|---|---|---|
| Berechnet: | 60,36 % | 9,03 % | 6,12 % | 10,51 % |
| Gefunden: | 58,30 % | 7,35 % | 6,08 % | 10,07 % |

1-[3-(1-Phenyl-2-methylsulfonyl-äthylthio)-2-D-methylpropanoyl]-L-prolin, Dicyclohexylammoniumsalz.

Fp.: 168°C (Zers.)

Analyse: $C_{30}H_{48}N_2O_5S_2$

| | C | H | N | S |
|---|---|---|---|---|
| Berechnet: | 62,04 % | 8,33 % | 4,82 % | 11,04 % |
| Gefunden: | 62,49 % | 8,41 % | 5,17 % | 10,48 % |

1-[3-(3-Oxo-n-butylthio)-2-D-methylpropanoyl]-L-prolin, Dicyclohexylammoniumsalz.

Fp.: 125°C

Analyse: $C_{25}H_{44}N_2O_4S$

| | C | H | N | S |
|---|---|---|---|---|
| Berechnet: | 64,06 % | 9,46 % | 5,98 % | 6,84 % |
| Gefunden: | 63,78 % | 9,53 % | 5,64 % | 7,58 % |

1-[3-(1-p-Tolyl-2-nitroäthylthio)-2-D-methylpropanoyl]-L-prolin.

Fp.: 58°C

Analyse: $C_{18}H_{24}H_2O_5S$

| | C | H | N | S |
|---|---|---|---|---|
| Berechnet: | 56,83 % | 6,36 % | 7,36 % | 8,43 % |
| Gefunden: | 56,33 % | 6,34 % | 7,03 % | 8,67 % |

## Pharmazeutische Anwendungsbeispiele

### a) Dragees

1 Drageekern enthält:

| | |
|---|---|
| Wirkstoff gemäß Anspruch 1 | 100,0 mg |
| Milchzucker | 60,0 mg |
| Maisstärke | 35,0 mg |
| Gelatine | 3,0 mg |
| Magnesiumstearat | 2,0 mg |
| | 200,0 mg |

**Herstellung:**

Die Mischung der Wirksubstanz mit Milchzucker und Maisstärke wird mit einer 10%igen wässrigen Gelatinelösung durch ein Sieb mit 1 mm Maschenweite granuliert, bei 40°C getrocknet und nochmals durch ein Sieb gerieben. Das so erhaltene Granulat wird mit Magnesiumstearat gemischt und verpreßt. Die so erhaltenen Kerne werden in üblicher Weise mit einer Hülle überzogen, die mit Hilfe einer wässrigen Suspension von Zucker, Titandioxyd, Talkum und Gummi arabicum aufgebracht wird. Die fertigen Dragees werden mit Bienenwachs poliert.

b) Tabletten

| | |
|---|---|
| Wirkstoff gemäß Anspruch 1 | 100,0 mg |
| Milchzucker | 70,0 mg |
| Maisstärke | 50,0 mg |
| lösliche Stärke | 7,0 mg |
| Magnesiumstearat | 3,0 mg |
| | 230,0 mg |

Herstellung:

Wirkstoff und Magnesiumstearat werden mit einer wässrigen Lösung der löslichen Stärke granuliert, das Granulat getrocknet und innig mit Milchzucker und Maisstärke vermischt. Das Gemisch wird sodann zu Tabletten von 230 mg Gewicht verpreßt, die je 100 mg Wirkstoff enthalten.

c) Injektionslösungen

| | | |
|---|---|---|
| Wirkstoff gemäß Anspruch 1 | | 50,0 mg |
| Äthanolamin | | 60,0 mg |
| Natriumchlorid | | 20,0 mg |
| destilliertes Wasser | ad | 2 ml |

Herstellung:

Der Wirkstoff und die Hilfsstoffe werden in einer ausreichenden Menge destilliertem Wasser gelöst und mit der erforderlichen Menge Wasser auf die gewünschte Konzentration gebracht. Die Lösung wird filtriert und unter aseptischen Bedingungen in 2 ml Ampullen abgefüllt. Die Ampullen werden sterilisiert und verschlossen. Jede Ampulle enthält 50 mg Wirkstoff.

d) Kapseln

| | |
|---|---|
| Wirkstoff gemäß Anspruch 1 | 100,0 mg |
| Milchzucker | 250,0 mg |
| Maisstärke | 40,0 mg |
| Talk | 10,0 mg |
| | 400,0 mg |

Wirkstoff, Milchzucker und Maisstärke werden zunächst in einem Mischer und dann in einer Zerkleinerungsmaschine vermengt. Das Gemisch wird nochmals in den Mischer gegeben, gründlich mit dem Talk vermengt und maschinell in Hartgelatinekapseln abgefüllt.

e) Suppositorien

| | |
|---|---|
| Wirkstoff gemäß Anspruch 1 | 0,1 g |
| Kakaobutter (Fp. 36-37°C) | 1,6 g |
| Carnaubawachs | 0,1 g |
| | 1,8 g |

Kakaobutter und Carnaubawachs werden geschmolzen, gründlich vermengt und auf 45°C abgekühlt. In diese Masse wird der feinpulverisierte Wirkstoff eingerührt. Anschließend wird die Mischung in leicht vorgekühlten Suppositorienformen geeigneter Größe gegossen und abkühlen gelassen.

## Patentansprüche

1. Substituierte Alkylthioacylaminosäuren der allgemeinen Formel

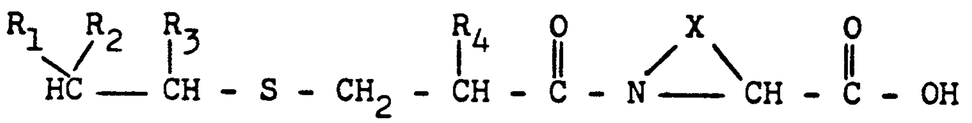 (I)

worin

$R_1$ die Nitro-, Cyano- oder Pyridylgruppe oder eine der Gruppen $- COR'$, $- COOR'$, $- PO(OR')_2$ oder

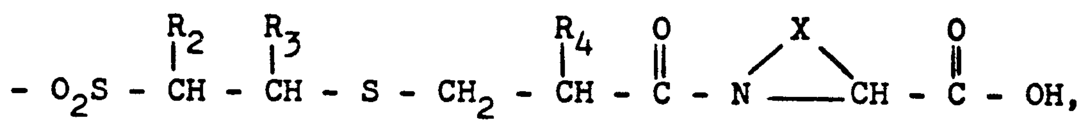

$R_2$ Wasserstoff, eine niedere Alkylgruppe mit 1 oder 2 Kohlenstoffatomen, eine der Gruppen $- COOR'$, $COR'$ oder den Rest $- SO_2CH_3$,

$R_3$ Wasserstoff, eine Alkoxygruppe mit 1 - 3 Kohlenstoffatomen, eine Phenylgruppe oder einen Thienylrest,

$R_4$ Wasserstoff oder die Methylgruppe,

X eine der Gruppen $- CH_2 - CH_2 - CH_2 -$ oder $- CH_2 - S - CH_2 -$ und

R' eine Alkylgruppe mit 1 oder 2 Kohlenstoffatomen bedeutet sowie deren Salze.

2. Verbindungen der allgemeinen Formel I gemäß Anspruch 1, worin
$R_1$ Wasserstoff, Acetyl oder Methoxycarbonyl,
$R_2$ Nitro, Acetyl oder Methoxycarbonyl,
$R_3$ Phenyl, Thienyl oder Methoxy,
$R_4$ Methyl und
X $-CH_2-CH_2-CH_2-$ oder $-CH_2-S-CH_2-$
bedeutet sowie deren Salze.

3. 1-[3-(1-Phenyl-2-nitro-äthylthio)-2-D-methylpropanoyl]-L-prolin und dessen Salze.

4. 1-[3-(1-Thienyl(2)-2-nitro-äthylthio)-2-D-methylpropanoyl]-L-prolin und dessen Salze.

5. 1-[3-(1-Phenyl-2-nitro-äthylthio)-2-D,L-methylpropanoyl]-L-thiazolidin-4-carbonsäure und dessen Salze.

6. 1-[3-(1-Methoxy-2,2-bis-{methoxycarbonyl}-äthylthio)-2-D-methylpropanoyl]-L-prolin und dessen Salze.

7. 1-[3-(2-Pyridyl(4)-äthylthio)-2-D-methylpropanoyl]-L-prolin und dessen Salze.

8. Verfahren zur Herstellung von Verbindungen der allgemeinen Formel I gemäß Anspruch 1, dadurch gekennzeichnet, daß man
a) die freie Mercaptogruppe einer Verbindung der allgemeinen Formel

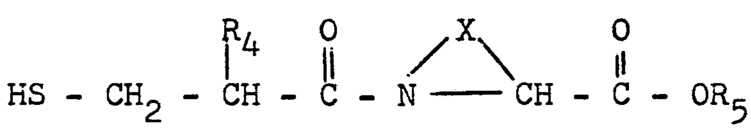

(II)

worin die Reste $R_4$ und X die oben angegebene Bedeutung haben und $R_5$ Wasserstoff oder eine niedere Alkylgruppe mit 1 - 3 Kohlenstoffatomen bedeutet, an die Doppelbindung eines durch mindestens eine elektronenanziehende Gruppe aktivierten Olefins der allgemeinen Formel

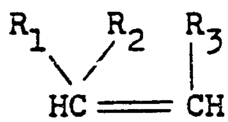
(III)

worin die Reste $R_1$, $R_2$ und $R_3$ die oben angegebene Bedeutung haben, addiert und, falls $R_5$ von Wasserstoff verschieden ist, die Estergruppe in saurem oder alkalischen Milieu abspaltet, oder daß man

b) eine substituierte Alkylthiocarbonsäure der allgemeinen Formel

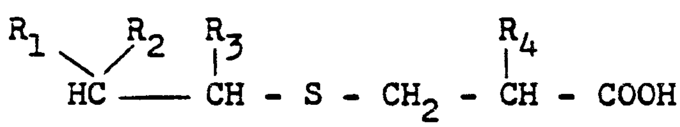
(IV)

worin die Reste $R_1$, $R_2$, $R_3$ und $R_4$ die oben erwähnte Bedeutung haben, mit einer Aminosäure der allgemeinen Formel

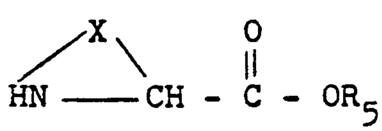
(V)

worin $R_5$ die oben erwähnte Bedeutung hat, umsetzt, eine etwa vorhandene Estergruppe durch hydrolytische oder acidolytische Spaltung entfernt und gegebenenfalls ein so erhaltenes Endprodukt der allgemeinen Formel I in ein Salz überführt.

9. Verfahren zur Herstellung dimerer Verbindungen der allgemeinen Formel I, worin $R_1$ die Gruppe

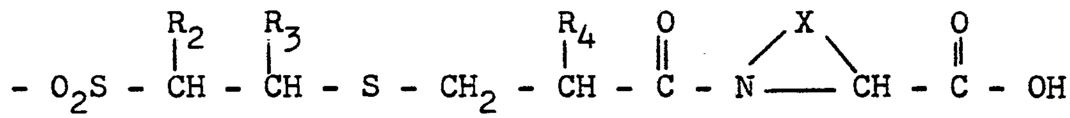

bedeutet, dadurch gekennzeichnet, daß man eine Verbindung der allgemeinen Formel II mit Divinylsulfon im Molverhältnis 2:1 umsetzt.

10. Pharmazeutische Zubereitungen, enthaltend als Wirkstoff eine oder mehrere Verbindungen der Formel I oder deren Salze in Kombination mit üblichen Hilfs- und/oder Trägerstoffen.

11. Pharmazeutische Zubereitungen, enthaltend als Wirkstoff eine oder mehrere Verbindungen der allgemeinen Formel I in Gemisch mit anderen bekannten Saluretica beziehungsweise Diuretica und/oder Antihypertonica.

12. Verfahren zur Herstellung von pharmazeutischen Präparaten gemäß Anspruch 10, dadurch gekennzeichnet, daß man Verbindungen der allgemeinen Formel I mit üblichen galenischen Hilfs- und/oder Trägerstoffen zu üblichen pharmazeutischen Anwendungsformen verarbeitet.

13. Verwendung der Verbindungen nach Anspruch 1 als Hypotensiva.

Europäisches Patentamt

**EUROPÄISCHER TEILRECHERCHENBERICHT,** der nach Regel 45 des Europäischen Patentübereinkommens für das weitere Verfahren als europäischer Recherchenbericht gilt

0036572

Nummer der Anmeldung
EP 81101819.1

## EINSCHLÄGIGE DOKUMENTE

| Kategorie | Kennzeichnung des Dokuments mit Angabe, soweit erforderlich, der maßgeblichen Teile | betrifft Anspruch |
|---|---|---|
| | DE - A1 - 2 703 828 (E.R. SQUIBB)<br>+ Patentansprüche 1,28,29 +<br>-- | 1,8,10 |
| | DE - A1 - 2 828 578 (YOSHITOMI)<br>+ Patentansprüche 1,29,30 +<br>-- | 1,8,10 |
| | DE - A1 - 2 842 100 (SCIENCE UNION)<br>+ Patentansprüche 1,7,8,10 +<br>-- | 1,8,10,11 |
| | DE - A1 - 2 930 605 (E.R. SQUIBB) (07-02-1980)<br>+ Patentanspruch 1; Seite 4, Zeilen 29-32; Seite 5, Zeilen 1-23 +<br>---- | 1,10 |

**KLASSIFIKATION DER ANMELDUNG (Int. Cl.[3])**

C 07 D 207/16
C 07 D 277/06
C 07 D 401/12
C 07 D 409/12
C 07 D 417/12
A 61 K 31/40
A 61 K 31/425
A 61 K 31/44

**RECHERCHIERTE SACHGEBIETE (Int. Cl.[3])**

C 07 D 207/00
C 07 D 277/00
C 07 D 401/00
C 07 D 409/00
C 07 D 417/00
A 61 K 31/00

## UNVOLLSTÄNDIGE RECHERCHE

Nach Auffassung der Recherchenabteilung entspricht die vorliegende europäische Patentanmeldung den Vorschriften des Europäischen Patentübereinkommens so wenig, daß es nicht möglich ist, auf der Grundlage einiger Patentansprüche sinnvolle Ermittlungen über den Stand der Technik durchzuführen.

Vollständig recherchierte Patentansprüche: 1-12
Unvollständig recherchierte Patentansprüche: -
Nicht recherchierte Patentansprüche: 13
Grund für die Beschränkung der Recherche:

Verfahren zur therapeutischen Behandlung des menschlichen oder tierischen Körpers, Artikel 52(4) EPÜ)

**KATEGORIE DER GENANNTEN DOKUMENTE**

X: von besonderer Bedeutung
A: technologischer Hintergrund
O: nichtschriftliche Offenbarung
P: Zwischenliteratur
T: der Erfindung zugrunde liegende Theorien oder Grundsätze
E: kollidierende Anmeldung
D: in der Anmeldung angeführtes Dokument
L: aus andern Gründen angeführtes Dokument
&: Mitglied der gleichen Patentfamilie, übereinstimmendes Dokument

| Recherchenort | Abschlußdatum der Recherche | Prüfer |
|---|---|---|
| WIEN | 22-06-1981 | ONDER |